(19) Europäisches Patentamt
European Patent Office
Office européen des brevets

(11) Veröffentlichungsnummer: **0 261 602**
**A2**

(12) **EUROPÄISCHE PATENTANMELDUNG**

(21) Anmeldenummer: **87113686.7**

(22) Anmeldetag: **18.09.87**

(51) Int. Cl.⁴: **C07D 213/38** , C07D 213/55 , C07D 213/57 , C07D 401/04 , B41M 5/12

(30) Priorität: **20.09.86 DE 3632009**

(43) Veröffentlichungstag der Anmeldung:
**30.03.88 Patentblatt 88/13**

(84) Benannte Vertragsstaaten:
**CH DE FR GB IT LI**

(71) Anmelder: **BASF Aktiengesellschaft**
**Carl-Bosch-Strasse 38**
**D-6700 Ludwigshafen(DE)**

(72) Erfinder: **Mayer, Udo, Dr.**
**Max-Slevogt-Strasse 27**
**D-6710 Frankenthal(DE)**
Erfinder: **Raulfs, Friedrich-Wilhelm, Dr.**
**Schulstrasse 9-17**
**D-6700 Ludwigshafen(DE)**

(54) **Pyridinverbindungen und deren Verwendung.**

(57) Es werden Pyridinverbindungen der allgemeinen Formel

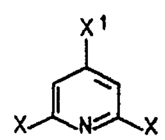

,

beschrieben, in der ein X für Phenyl und das andere X für durch Halogen, Cyano, $C_1$-bis $C_5$-Alkyl, $C_1$-bis $C_5$-Alkoxy oder $C_1$-$C_5$-Alkoxycarbonyl substituiertes Phenyl oder beide X für 4-Fluorphenyl und $X^1$ für einen der Reste der Formeln

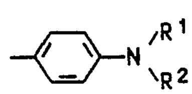

oder

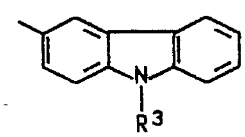

stehen,
worin
$R^1$ und $R^2$ unabhängig voneinander lineares oder verzweigtes Akyl, Cyanalkyl, Halogenalkyl, Aryl oder Aralkyl mit insgesamt 7 bis 14 C-Atomen oder

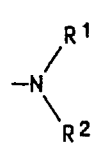

EP 0 261 602 A2

einen 5-oder 6-gliedrigen gesättigten heterocyclischen Rest und

$R^3$ lineares oder verzweigtes $C_1$-bis $C_4$-Alkyl bedeuten.

Die Verbindungen (I) sind Farbbildner für druckempfindliche Aufzeichnungssysteme.

## Pyridinverbindungen und deren Verwendung

Aus der DE-A 2 227 597 sind symmetrische Pyridinverbindungen bekannt, die in 2-und 6-Stellung Phenyl-und p-Methoxyphenylreste enthalten.

Für die Anwendung in einem photographischen Kopierverfahren wird in der GB-A 2 029 591 ein Farbbildner beschrieben, der in 2-und 6-Stellung 4-Chlorphenyl enthält.

Aminosubstituierte Phenylringe in 2-und 6-Stellung des Pyridinrings sind aus der EP-A-061 128 bekannt.

Diese Farbbildner eignen sich für druckempfindliche Aufzeichnungssysteme, haben jedoch bei der Anwendung Nachteile, z.B. zu geringe Löslichkeit, zu starke Migration und zu starke Anfärbung von Streichrohpapier.

Aufgabe der vorliegenden Anmeldung war es, bezüglich der Löslichkeit, Migration und Streichrohpapieranfärbung verbesserte Farbbildner bereitzustellen.

Die Erfindung betrifft Pyridinverbindungen der allgemeinen Formel (I)

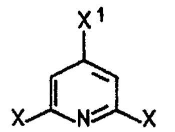

(I),

in der ein X für Phenyl und das andere X für durch Halogen, Cyano, $C_1$-bis $C_5$-Alkyl, $C_1$-bis $C_5$-Alkoxy oder $C_1$-$C_5$-Alkoxycarbonyl ein-oder zweifach substituiertes Phenyl oder beide X für 4-Fluorphenyl und $X^1$ für einen Rest der Formel

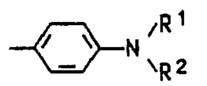

oder

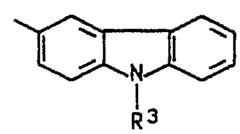

,

(II) (III)

stehen, worin

$R^1$ und $R^2$ unabhängig voneinander lineares oder verzweigtes $C_1$-$C_8$-Alky l, Cyan-$C_2$-$C_5$-alkyl, Halogen-$C_1$-$C_5$-alkyl, Aryl oder Aralkyl mit insgesamt 7 bis 14 C-Atomen oder

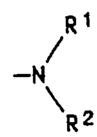

einen 5-oder 6-gliedrigen gesättigten heterocyclischen Rest und

$R^3$ lineares oder verzweigtes $C_1$-bis $C_4$-Alkyl bedeuten.

Die Pyridinverbindungen sind schwach gelbe bis farblose Verbindungen. In Eisessig gelöst erhält man gelbe bis orange Färbungen. In neutralen oder basischen organischen Lösungsmitteln erhält man farblose Lösungen. Die Farbbildung kann auch durch Kaolin, Zeolithe, Bentonit, Kieselsäure, Alaun, Zinksulfat, Oxalsäure und phenolische Kondensationsprodukte bewirkt werden. Durch diese Eigenschaft eignen sich die Verbindungen als Farbbildner für druckempfindliches Aufzeichnungsmaterial, insbesondere für die Herstellung von Kopierpapieren.

Im Vergleich zu den gelbe bis orange Färbungen liefernden Pyridinderivaten des Standes der Technik migrieren die Pyridinverbindungen (I) weniger. Auch färben die Verbindungen (I) Streichrohpapier sowohl bei Raumtemperatur als auch bei 150 °C weniger an. Einige Pyridinverbindungen (I) sind in den Anwendungsmedien besser löslich als die Pyridinderivate des Standes der Technik.

Das eine X steht für Phenyl. Das andere X steht für durch Halogen, Cyano, $C_1$-bis $C_5$-Alkyl, $C_1$-bis $C_5$-Alkoxy oder $C_1$-$C_5$-Alkoxycarbonyl substituiertes Phenyl oder beide X stehen für 4-Fluorphenyl. Halogen ist Brom, vorzugsweise Chlor oder Fluor. Die Zahl der Substituenten an diesem X ist eins oder zwei, vorzugsweise eins, bei Chlor eins oder zwei.

Für das andere X sind im einzelnen zu nennen: 3-und 4-Methylphenyl, 2-und 4-Methoxyphenyl und 4-Chlorphenyl, 4-Bromphenyl, 4-Fluorphenyl, 2-Carboethoxyphenyl, 4-Cyanphenyl und 3,4-Dichlorphenyl von denen 4-Fluorphenyl und insbesondere 4-Chlorphenyl bevorzugt sind.

$X^1$ steht für den Rest der Formel (II) oder (III). In (II) stehen $R^1$ und $R^2$ unabhängig voneinander für lineares, verzweigtes oder cyclisches $C_1$-bis $C_8$-Alkyl, wie Methyl-, Ethyl-, Propyl, iso-Propyl, Butyl, iso-Butyl, tertiär Butyl, Hexyl, Cyclohexyl, 2-Ethylhexyl und Octyl, für Cyan-$C_2$-$C_5$-alkyl wie 2-Cyanethyl, für Halogen-$C_1$-$C_5$-alkyl wie 2-Chlorethyl, 2-Bromethyl, 2-Chlorpropyl, 3-Brompropyl und 4-Chlorbutyl.

Aryl steht vorzugsweise für gegebenenfalls substituiertes Phenyl und Aralkyl für Phenalkyl mit insgesamt 7 bis 14 C-Atomen, z.B. p-Xylyl, m-Xylyl, o-Xylyl, Cumyl, 1-Phenylhexyl und und Benzyl, 2-Phenylethyl.

Für $R^1$ und $R^2$ sind Methyl, Ethyl, Butyl, 2-Cyanethyl, 2-Chlorethyl und Benzyl bevorzugt.

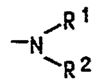

kann außerdem für den Rest eines 5-oder 6-gliedrigen gesättigten heterocyclischen Ringes stehen, z.B. für Piperidinyl, Morpholinyl, Piperazinyl, Pyrrolidinyl oder N'-$C_1$-bis $C_4$-Alkylpiperazinyl. In (III) steht $R^3$ für $C_1$-bis $C_4$-Alkyl, vorzugsweise für Methyl oder Ethyl.

Vorzugsweise steht $X^1$ für 4-N-Benzyl-N-ethyl-aminophenyl, 4,N-(2'-Cyanethyl)-N-butyaminophenyl, 4-N-(2'-Cyanethyl)-N-methylaminophenyl, insbesondere für 4-N,N-Dimethylaminophenyl.

Die Pyridinverbindungen (I) erhält man zum Beispiel durch Umsetzen von Vinylketonen der Formel (IV)

$X-\underset{\overset{\|}{O}}{C}-CH=CH-X^1$ (IV)

mit quartären Ammoniumsalzen der allgemeinen Formel (V)

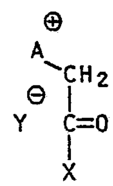

(V)

in Gegenwart von Ammoniak oder Ammoniak abspaltenden Mitteln in Essigsäure.

In der Formel (V) bedeutet A entweder eine Ammoniumgruppe der Formel (VI)

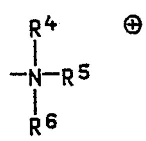

(VI),

in der $R^4$, $R^5$ und $R^6$ unabhängig voneinander für lineares oder verzweigtes $C_1$-bis $C_8$-Alkyl, und $R^4$ und $R^5$ zusammen mit dem N-Atomen der Ammoniumgruppe auch für einen gesättigten 5-oder 6-gliedrigen heterocyclischen Ring stehen, oder einen Pyridiniumrest der Formel (VII)

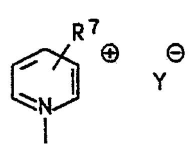

(VII),

worin $R^7$ Wasserstoff oder ein lineares oder verzweigtes $C_1$-bis $C_4$-Alkyl und $Y^{\ominus}$ ein organisches oder anorganisches Anion ist. Vorzugsweise steht $Y^{\ominus}$ für Chlorid oder Bromid.

Die Erfindung soll durch die folgenden Beispiele zusätzlich erläutert werden.

Die in den Ausführungsbeispielen genannten Teile und Prozente beziehen sich auf das Gewicht.

Beispiel 1

10,06 Teile 1-(4'-Fluorphenacyl)-pyridiniumchlorid und 10,76 Teile 1-(4-Fluorphenyl)-3-(4'-dimethylaminophenyl)-1-oxoprop-2-en werden zusammen mit 34 Teilen Ammoniumacetat in 50 Teilen Eisessig 8 ½ Stunden bei 130 °C erhitzt. Nach 17-stündigem Rühren bei Raumtemperatur werden die ausgefallenen farblosen Kristalle abgesaugt, mit Methanol und Wasser gewaschen und bei 60 °C im Trockenschrank getrocknet. Man erhält 10,5 Teile 4-(4-Dimethylaminophenyl)-2,6-bis(4"-fluorphenyl)pyridin, welches sich in Eisessig mit einer orangegelben Farbe löst (λmax: 431 nm).

Beispiel 2

Es wird nach der gleichen Vorschrift wie unter Beispiel 1 verfahren, jedoch werden statt 10,06 Teilen 1-(4-Fluorphenacyl)-pyridiniumchlorid 10,94 Teile (4-Fluorphenacyl)triethylammoniumchlorid eingesetzt. Die Ausbeute nach 13-stündigem Erhitzen auf 120 °C beläuft sich auf 2,3 Teile 4-(4-Dimethylaminophenyl)-2,6-bis(4-fluorphenyl)pyridin.

Beispiel 3

13,9 Teile 1-Phenacylpyridiniumbromid und 14,27 Teile 1-(4-Chlorphenyl)-3-(4-dimethylaminophenyl)-1-oxoprop-2-en werden zusammen mit 34 Teilen Ammoniumacetat in 50 Teilen Eisessig 3,5 Stunden auf 120 °C erhitzt. Nach der Aufarbeitung gemäß Beispiel 1 erhält man 4-(4'-Dimethylaminophenyl)-2-(4'-chlorphenyl)-6-phenylpyridin in Form eines farblosen Pulvers, das sich in Eisessig mit gelber Farbe löst (λmax: 425 nm).

Beispiele 4 bis 15

Analog den Beispielen 1 bis 3 wurden die folgenden Pyridinverbindungen (I) hergestellt:

| Beispiel | $X^2$ | $X^1$ | $X^3$ | λ max [nm] |
|---|---|---|---|---|
| 4 | Phenyl | 4-Dimethylaminophenyl | 2-Methoxy-phenyl | 436 |
| 5 | Phenyl | 4-Dimethylaminophenyl | 4-Methyl-phenyl | 425 |
| 6 | Phenyl | 4-Dimethylaminophenyl | 3-Methyl-phenyl | 430 |
| 7 | Phenyl | 4-Dimethylaminophenyl | 4-Fluor-phenyl | 425 |
| 8 | Phenyl | 4-(2-Cyanethyl)-methyl-aminophenyl | 4-Chlor-phenyl | 425 |
| 9 | Phenyl | 4-Butyl(2-cyanethyl)-aminophenyl | 4-Chlorphenyl | 428 |
| 10 | Phenyl | 9-Ethylcarbazol-3-yl | 4-Chlorphenyl | 407 |
| 11 | Phenyl | 4-Benzylethyl-amino-phenyl | 4-Chlorphenyl | 439 |
| 12 | Phenyl | 4-(2-Chlorethyl)-ethylaminophenyl | 4-Chlorphenyl | 436 |
| 13 | Phenyl | 4-Dimethylaminophenyl | 2-Carboethoxypenyl | 433 |
| 14 | Phenyl | 4-Dimethylaminophenyl | 4-Cyanphenyl | 444 |
| 15 | Phenyl | 4-Dimethylaminophenyl | 3,4-Dichlorphenyl | 442 |

Beispiel 16

9,9 Teile 3-Methyl-1-phenacylpyridiniumchlorid, 11,4 Teile 1-(4-Chlorphenyl)-3-(4-dimethyl-aminophenyl)-1-oxoprop-2-en und 34 Teile Ammoniumchlorid werden in 50 Teilen Eisessig 2,5 Stunden unter Rückfluß erhitzt. Aus der roten Lösung fallen nach einer Stunde weiße Kristalle von 2-(4'-Chlorphenyl-4-(4'-dimethylaminophenyl)-6-phenylpyridin aus. Nach 17-stündigem Rühren bei Raumtemperatur wird abgesaugt mit Methanol und Wasser gewaschen und bei 60 °C getrocknet. Die Ausbeute beläuft sich auf 12 Teile Produkt, das mit den nach Beispiel 3 erhaltenen identisch ist.

## Ansprüche

1. Pyridinverbindungen der allgemeinen Formel

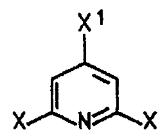

in der ein X für Phenyl und das andere X für durch Halogen, Cyano, $C_1$-bis $C_5$-Alkyl, $C_1$-bis $C_5$-Alkoxy oder $C_1$-$C_5$-Alkoxycarbonyl ein-oder zweifach substituiertes Phenyl oder beide X für 4-Fluorphenyl und $X^1$ für einen Rest der Formel

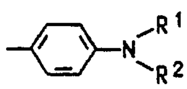

oder

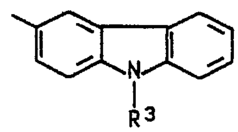

,

stehen, worin

$R^1$ und $R^2$ unabhängig voneinander lineares oder verzweigtes $C_1$-$C_8$-Alkyl, Cyan-$C_2$-$C_5$-Alkyl, Halogen-$C_1$-$C_5$-alkyl, Aryl oder Aralkyl mit insgesamt 7 bis 14 C-Atomen oder

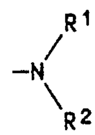

$R^3$ einen 5-oder 6-gliedrigen gesättigten heterocyclischen Rest und lineares oder verzweigtes $C_1$-bis $C_4$-Alkyl bedeuten. .

2. Pyridinverbindungen gemäß Anspruch 1, dadurch gekennzeichnet, daß das andere X für 4-Fluorphenyl, 4-Chlorphenyl, 4-Methylphenyl, 4-Methoxyphenyl, 2-Methoxyphenyl, 2-Carboethoxyphenyl, 4-Cyanphenyl oder 3,4-Dichlorphenyl steht.

3. Pyridinverbindungen gemäß den Ansprüchen 1 oder 2, dadurch gekennzeichnet, daß $X^1$ für 4-N,N-Dimethylaminophenyl, 4-N-(2'-Cyanethyl)-N-methylaminophenyl, 4-N-(2'-Cyanethyl)-N-butylaminophenyl, 4-N-Benzyl-N-ethylaminophenyl, 4-N-(2'-Chlorethyl)-N-ethylaminophenyl oder 9-Ethylcarbazol-3-yl steht.

4. Pyridinverbindungen gemäß Anspruch 1 oder 2, dadurch gekennzeichnet, daß $X^1$ für 4-N-Benzyl-N-ethylaminophenyl, 4-N-(2'-Cyanethyl)-N-butylaminophenyl, 4-(N-(2'-Cyanethyl)-N-methylaminophenyl oder für 4-N,N-Dimethylaminophenyl steht.

5. Pyridinverbindungen gemäß Anspruch 11 oder 2, dadurch gekennzeichnet, daß $X^1$ für 4-N,N-Dimethylaminophenyl steht.

6. Pyridinverbindung gemäß Anspruch 1, gekennzeichnet durch die Formel

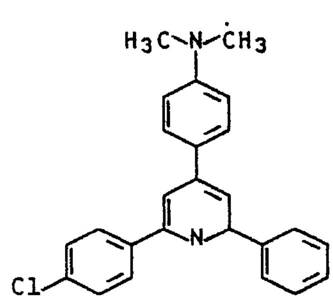

7. Verwendung der Pyridinverbindungen gemäß den Ansprüchen 1 bis 6 als Farbbildner in Druckempfindlichen Aufzeichnungssystemen.-